# EUROPEAN PATENT APPLICATION

(11) **EP 4 682 821 A1**
(43) Date of publication of application: **21.01.2026**
(21) Application number: 24189520.0
(22) Date of filing: 18.07.2024
(51) Int. Cl.: G06T 7/00, G16H 50/20

(54) **METHOD OF ANALYZING 3D DATA ACQUIRED BY A 3D IMAGE-PRODUCING PROCEDURE IN AN AREA OF INSPECTION, COMPUTER-BASED CLINICAL DECISION SUPPORT SYSTEM, COMPUTER PROGRAM PRODUCT AND COMPUTER-READABLE MEDIUM**

(71) Applicant: OLYMPUS WINTER & IBE GMBH, 22045 Hamburg (DE)
(72) Inventor: ENDERS, Borg, 21149 Hamburg (DE)
(74) Representative: Seemann & Partner Patentanwälte mbB

(57) **Abstract**

The method of analyzing 3D data (D) acquired by a 3D image-producing procedure in an area of inspection comprising at least one body part of a patient's body comprises the steps of:
- receiving the 3D data (D),
- determining a 3D model (M) of the body part from the 3D data (D) by inputting the 3D data (D) into an artificial intelligence model (8) to perform an inference operation based on the 3D data (D) to generate the 3D model (M) of the body part and a classifier (C), which is indicative of at least one anomaly in the 3D model (M) of the body part,
- outputting the 3D model (M) together with the classifier (C) at a user interface (10).

## Description

The invention relates to a method of analyzing 3D data acquired by a 3D image-producing procedure in an area of inspection comprising at least one body part of a patient's body. Furthermore, the invention relates to a computer-based clinical decision support system (CDSS) for executing this method. The invention also relates to a computer program product and to a computer-readable medium.

3D image-producing procedures, for example computer tomography (CT) or magnetic resonance imaging (MRI), produce a high amount of image data or data from which images can be derived. This data is investigated manually by a radiologist or surgeon. Additionally, the data can be processed to generate a 3D model. In this process, the specific anatomy that is relevant for a surgical procedure is typically taken into account. In other words, the 3D model is rendered to a specific part of the body, for example on an inner organ like the liver, kidney or lung, which is subject to later surgical treatment. The 3D data comprises a large amount of information. During review of the data, the radiologist or surgeon is focused on the single pathology or on a single body part. This is done to efficiently analyze the data. The necessity to focus on relevant parts of the data can however result in a situation, in which anomalies in currently not deemed relevant parts of the data are not recognized.

A similar situation can occur during generation of the 3D model. In an attempt to focus on the most relevant information, the 3D model is typically rendered only on the body part that seems to be directly related to for example a surgical procedure. If for example the surgical procedure is planned for the liver or kidney, only the part of the 3D data imaging this organ is rendered on a 3D model. The 3D model can later be used to assist the surgeon during execution of the surgical procedure on this particular inner organ. Due to this approach, a high amount of additional information in the 3D data is not analyzed or even considered. However, the additional information in the 3D data can be relevant or helpful for the surgical procedure.

It is an object of the invention to provide a method of analyzing 3D data acquired by 3D image producing procedures, a computer-based clinical decision support system for execution of this method, a computer program product and a computer-readable medium, that are enhanced with respect to the deficiencies in the prior art.

The object is solved by a method of analyzing 3D data acquired by a 3D image-producing procedure in an area of inspection comprising at least one body part of a patient's body, the method comprising the steps of:
- receiving the 3D data,
- determining a 3D model of the body part from the 3D data by inputting the 3D data into an artificial intelligence model to perform an inference operation based on the 3D data to generate the 3D model of the body part and a classifier, which is indicative of at least one anomaly in the 3D model of the body part,
- outputting the 3D model together with the classifier at a user interface.

The 3D data is in particular acquired by magnetic resonance imaging (MRI) and/or computer tomography (CT) by scanning of a patient's body in the area of inspection. The method of analyzing the 3D data according to aspects of the invention can comprise the step of acquiring the 3D data. In another aspect, the step of acquiring the 3D data does not form part of the method of analyzing the 3D data according to aspects of the invention. The method of analyzing the 3D data can be a computer-implemented method.

The method according to aspects of the invention can be applied to assist a person inspecting the 3D data, for example a radiologist or surgeon. The information resulting from the method can be used or applied for preparation of a surgical procedure. Furthermore, the information resulting from the method can be used or applied during a surgical procedure. The method can comprise the step of conducting the surgical procedure. In another aspect, the method according to aspects of the invention does not comprise the step of conducting the surgical procedure. The surgical procedure can be conducted subsequent to the method of analyzing the 3D data, i.e. after the termination of the method.

The classifier helps the person inspecting the 3D data. As mentioned before, radiologists or surgeons are traditionally focused on the specific anatomical structure or body part, which can be an inner organ of the patient, that is relevant for the surgical procedure. The use of 3D models limited to a single organ emphasizes this very focused approach. It will also increase the risk to overlook other findings. Even earlier in the technical development, which means without the application of single organ 3D models, a radiologist at least superficial inspected all image data, which is a time-consuming task. In view of the high amount of data that is acquired for example during computer tomography (CT) or magnetic resonance imaging (MRI), these traditional approaches, however, inevitably lead to the situation that information that is potentially relevant for the surgical procedure is not inspected.

In contrast to this, with the method of analyzing the 3D data according to aspects of the invention, it is possible to analyze the full data set without spending excessive time for this analysis. The surgeon or radiologist can conduct the work as usual and focus on that part of the dataset that is relevant for the later surgical procedure. The remaining data can be analyzed in an automatic process or in an assisted process by applying the method according to aspects of the invention on this part of the data. According to an aspect of the invention, the method is applied on the full data set. Based on the classifier, the person inspecting the 3D data can decide to take further information into account. The classifier provides a suitable basis of decision-making and helps to also focus on for example more distant structures that would have been overlooked when solely focusing on the target area or body part.

According to an advantageous embodiment, the method is enhanced in that the artificial intelligence model is a neural network, being trained on 3D training data, for which at least one of the following criteria applies:
a) the training data describes standard anatomy models of the at least one body part,
b) the training data describes standard anatomy models of the at least one body part having deviations from standard anatomy by less than a predetermined deviation threshold,
wherein a degree of anomaly indicated by the classifier is a function of a deviation occurring when rendering the 3D model of the body part against the training data.

The training data can be taken from literature or from a suitable dataset reflecting standard anatomy. The training data can also be taken from real clinical findings or examinations, which have been performed with 3D imaging image-producing procedures. Only that data is used as training data, which comprises clinical findings that are considered clinically unremarkable. In other words, training data always shows a healthy situation. The MRI and/or CT data sets, which can be used as training data, show deviations from standard text book anatomy. However, the deviation is by then a predetermined deviation threshold. In other words, only 3D data sets showing body parts that comply with standard anatomy to a certain degree considered healthy, are taken as training data. The approach to also train the model on real data enriches the data pool representing standard anatomy and enhances the training of the neural network. The data used as a reference is hence the standard anatomy model, which can be supplemented by real world anatomy data showing a range of common but clinically uncritical anatomical variations. The neural network can be trained by supervised or unsupervised learning.

The artificial intelligence model can perform a rendering process to check the 3D data against the standard anatomy model or against an autonomy model showing potential ranges of common variations. For this check, the artificial intelligence model can be first trained to efficiently detect anomalies with respect to standard anatomy and to identify potentially relevant findings. The findings are indicated by the classifier. For this purpose, the artificial intelligence model can be trained with the data sets that are most similar to standard textbook anatomy. The artificial intelligence model, which is for example an algorithm or a neural network, can be trained to flag all deviations from the training data set. In a second step, the artificial intelligence model can be trained to use the 3D data set taken from real world 3D imaging, for example CT and/or MRI data showing real world healthy body parts or inner organs of patients. This data enriches the data pool of the artificial intelligence model.

The method can be further enhanced in that the artificial intelligence model is trained to perform the inference operation in that
a) the classifier indicates top priority, if the 3D model of the body part is not found in standard anatomy,
b) the classifier indicates medium priority, if at least one anatomy deviation of the 3D model from the standard anatomy is found, wherein this deviation is in a predetermined deviation interval,
c) the classifier indicates low priority, if an anatomy deviation of the 3D model from the standard anatomy is found, wherein this deviation is below a lower limit of the predetermined deviation interval.

The classifier helps or assists the person inspecting the 3D data to put focus on those parts of the data that would have been overlooked in traditional analysis. Regions or body parts with deviations from standard autonomy can be given a relevance flag, which is one example for the classifier. The person can then put further attention on this area.

The top priority elements are not found in standard anatomy. This covers for example lesions and other significant anatomy variations. The medium priority anatomy variations are near to what can be found in standard anatomy. However, there is a deviation, which is within a certain range of deviations, i.e. in the predetermined deviation interval. This interval covers deviations in shape from standard anatomy, for example small lesions. The deviation interval also comprises structures that can be found in standard anatomy (which means that there is no or little deviation in shape) but deviate in position. In other words, the detected structure, for example an organ can be found not in the exact same location as in standard anatomy. The low priority covers anatomy deviations which are below a lower limit of the above mentioned predetermined deviation interval. Hence, deviations which are not covered by class a) or b) are classified in class c).

According to still another advantageous embodiment, the artificial intelligence model further performs the inference operation based on the 3D data to generate the classifier, in that it is additionally indicative of a type of the at least one anomaly in the 3D model of the body part. The anomaly can be for example a lesion. Different lesions can be classified as different types of lesions.

The embodiment can be further enhanced in that the anomalies are prioritized on the anomaly list, wherein in particular the artificial intelligence model is further trained to perform an inference operation based on the 3D model to generate the classifier, in that it is additionally indicative of a relevance of the anomaly, based on which the prioritization on the anomaly list is performed, and wherein further in particular the classifier of every found anomaly comprises a relevance value.

The list of deviations can help the person inspecting the 3D data to perform the prioritization of the findings. The prioritization can be performed based on classification of the navigation, wherein the deviation can be classified using one or more following parameters: an anomaly's size, an anomaly's type of lesion and/or an amount of the anomaly's deviation from standard anatomy.

Furthermore, the method can be enhanced in that the anomaly list is displayed in a step-by-step procedure with one anomaly at a time. A region of the rendered 3D model corresponding to the respective anomaly can be highlighted and/or zoomed-in. In particular, additional information characterizing the respective anomaly can be added by user input. For easier review, the method can implement viewer function (for example by a software module) that allows to step through the deviations. This can be performed before the beginning of the surgical procedure. The radiologist or surgeon can for example classify the deviation and can decide to focus on particular deviations. The viewer function can highlight and zoom-in each area for a quick review. Furthermore, the person inspecting the 3D data, during this review, can add information to the deviations by inputting user content. This user comments can be added to the data set or included in the data set for later use, for example during a surgical procedure. With this, the data set is not only the direct 3D information of the body part but also includes user information and content.

The method can be further enhanced in that the area of inspection comprises a first area and a second area, wherein the first and the second area are not identical, and a first body part is located in the first area and a second body part is located in the second area, and the 3D data comprises information on the first and the second body part, wherein the first area and the first body part are labeled a subject and area of surgical procedure, and wherein a first 3D model is determined for the first body part and a second 3D model is determined for the second body part and at least the second 3D data of the second body part is input in the artificial intelligence model (8) to generate the second 3D model of the second body part and the first and second 3D model together with the classifier, which is indicative of at least one anomaly in the second 3D data of the second body part are output via the user interface.

According to still another advantageous embodiment, this method can be further enhanced by the step of outputting a prioritization information together with the classifier, the prioritization information indicating a distance between the first body part and the second body part, in particular the prioritization information indicates a distance between the first body part and an anomaly detected in the second body part.

The prioritization information that can be added to the data can include information on a distance of the found anomaly from the primary area of investigation, which can be the area, in which surgical procedure is conducted. This information can assist the surgeon during planning or execution of the later surgical procedure.

Furthermore, the object is solved by a computer-based clinical decision support system (CDSS), comprising a processing unit, a user interface and a display, configured to perform the method according to one or more of the previously mentioned aspects.

Same or similar advantages, which have been mentioned for the method of analyzing the 3D data, apply for the CDSS in a same or similar way and shall therefore not be repeated.

The object is also solved by a computer program product comprising instructions which, when the program is executed by a computer, cause the computer to perform the method according to one or more of the previously mentioned aspects.

The object is furthermore solved by a computer-readable medium comprising instructions which, when executed by a computer, cause the computer to perform the method according to one or more of the previously mentioned aspects.

Also the computer program and the computer-readable medium offer same or similar advantages, which have been mentioned with respect to the method according to aspects of the invention.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:

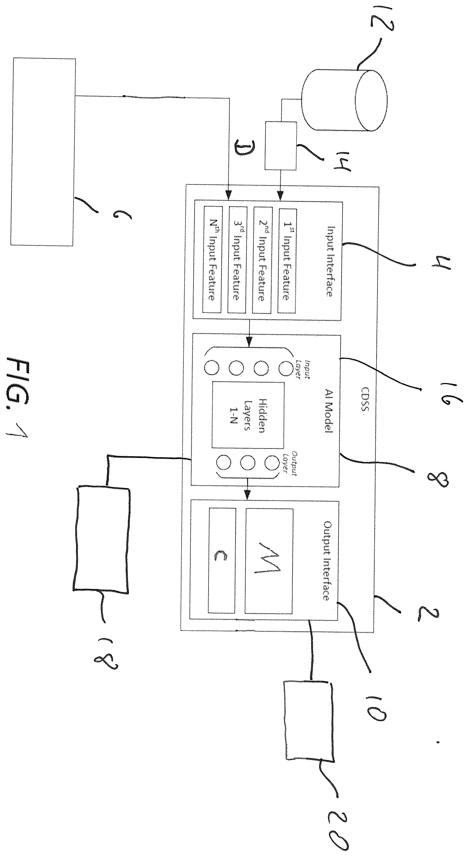

- Fig. 1: a clinical decision support system (CDSS), which is configured to implement the method of analyzing 3D data acquired by the 3D image processing procedure in an area of inspection.

In the drawings, the same or similar types of elements or respectively corresponding parts are provided with the same reference numbers in order to prevent the item from needing to be reintroduced.

Fig. 1 shows a schematic diagram of an exemplary computer-based clinical decision support system (CDSS) 2 that is configured to output a 3D model M of a body part, for example a 3D model M of an inner organ of a patient. The system 2 outputs the 3D model together with a classifier C that indicates at least one anomaly in the 3D data D or 3D model M of the body part. The 3D data D is input into the CDSS 2 via an input interface 4. The 3D data D is generated by the 3D image-producing procedure, which is for example computer tomography (CT) and/or magnetic resonance imaging (MRI). For this purpose, the CDSS 2 can include a 3D image-producing device 6, which is for example a computer tomograph or an MRI machine.

In various embodiments, the CDSS 2 includes the input interface 4 through which the patient specific 3D data D is provided as input features to an artificial intelligence (Al) model 8 of a processor 16, which performs an inference operation in which the 3D data D is applied to the AI model 8 to generate the 3D model M and the classifier C. This information is output via an output interface 10 to for example a display 20. The display 20 is the user interface of the CDSS 2, through which the 3D model M and the classifier C are communicated to a user, e.g., a clinician. The output can serve as the basis for planning of a surgical procedure. Furthermore, the output can also be used during a surgical procedure. The AI model 8 receives the plurality of input features via the input interface 4. The individual input features are represented by the image information of the 3D data D. CT or MRI data comprise a huge amount of image information, which means that the AI model 8 receives a high number of input features. The input features are processed by the input layers of the AI model 8 and a plurality of hidden layers. The data is processed for example through a neural network, which can be the AI model 8, to the output layers thereof. The data of the output layers is forwarded to the output interface 10.

In some embodiments, the input interface 4 may be a direct data link between the CDSS 2 and the one or more 3D image-producing devices 6 that generate at least some of the input features, which means the 3D data D. For example, the input interface 4 may transmit the 3D data D directly to the CDSS 2 during a therapeutic and/or diagnostic medical procedure. Additionally, or alternatively, the input interface 4 may be a classical user interface that facilitates interaction between a user and the CDSS 2. For example, the input interface 4 may facilitate a user interface through which the user may manually enter 3D data D, for example using a portable data storage.

Additionally, or alternatively, the input interface 4 may provide the CDSS 2 with access to a database 12, which inter alia holds an electronic patient record 14 from which the 3D data D may be extracted. In any of these cases, the input interface 4 is configured to collect the 3D data D in association with specific patient data on or before a time at which the CDSS 2 is used to generate the 3D model M and the classifier C.

Based on the 3D data D, the processor 16 holding the AI model 8 performs the inference operation using the AI model 8 to generate the 3D model M and the classifier C. For example, the input interface 4 receiving the 3D data D may deliver the CT or MRI data into the input layer of the AI model 8 which propagates this image data as input features through the AI model 8 to the output layer. The AI model 8 can provide a computer system with the ability to perform tasks, without explicitly being programmed, by making inferences based on patterns found in the analysis of data. The AI model 8 explores the study and construction of algorithms (e.g., machine-learning algorithms) that may learn from existing data, which is training data that will be explained in detail further below, and make predictions about new data. Such algorithms operate by building an AI model 8 from example training data in order to make data-driven predictions or decisions expressed as outputs or assessments.

There are two common modes for machine learning (ML): supervised ML and unsupervised ML. Supervised ML uses prior knowledge (e.g. examples that correlate inputs to outputs or outcomes) to learn the relationships between the inputs and the outputs. The goal of supervised ML is to learn a function that, given some training data, best approximates the relationship between the training inputs and outputs so that the ML model can implement the same relationships when given inputs to generate the corresponding outputs. Unsupervised ML is the training of an ML algorithm using information that is neither classified nor labeled, and allowing the algorithm to act on that information without guidance. Unsupervised ML is useful in exploratory analysis because it can automatically identify structure in data.

The AI model 8 of the CDSS 2 is for example a neural network, being trained on 3D training data, for which at least one of the following criteria applies: The training data describes standard anatomy models of the at least one body part. This data can for example be taken from literature. The training data can describe standard anatomy models of the at least one body part having deviations from standard anatomy by less than a predetermined deviation threshold. In other words, the training data does not exactly comply with textbook literature but allows for certain deviations. The training data can also be captured in real world measurements in a 3D image producing procedure like CT or MRI. This real world data shows healthy body parts and enriches the database or training data of the AI model 8 by further variations which often occur in practice. The training is performed in view of the 3D model M and the output classifier C. The degree of anomaly indicated by the output classifier C is a function of a deviation occurring when rendering the 3D model M of the body part against the training data.

The training of the AI model 8 is further conducted in that the AI model 8 is trained to perform the inference operation so the classifier C indicates top priority, if the 3D model M of the body part is not found in standard anatomy. Furthermore, in the training, the AI model 8 is configured in that the classifier C indicates medium priority, if at least one anatomy deviation of the 3D model M from the standard anatomy is found, wherein this deviation is within a predetermined deviation interval. Finally, the AI model 8 is trained so as to set the classifier C to low priority, if a deviation is detected for which neither the first nor the second case applies. The classifier C with low priority indicates anatomy deviations of the 3D model M from the standard anatomy, wherein the deviation is below a lower limit of the predetermined deviation interval.

The AI model 8 can be further trained to perform the inference operation based on the 3D data D in that the classifier C is additionally indicative of a type of the anomaly of the body part that is found in the 3D data D. For example, the classifier C can describe a type of lesion.

Common tasks for supervised ML are classification problems and regression problems. Classification problems, also referred to as categorization problems, aim at classifying items, in this case anomalies, into one of several category values (for example, is this object an apple or an orange?). Regression algorithms aim at quantifying some items (for example, by providing a score to the value of some input). Some examples of commonly used supervised ML algorithms are Logistic Regression (LR), Naive-Bayes, Random Forest (RF), neural networks (NN), deep neural networks (DNN), matrix factorization, and Support Vector Machines (SVM).

Some common tasks for unsupervised ML include clustering, representation learning, and density estimation. Some examples of commonly used unsupervised ML algorithms are K-means clustering, principal component analysis, and autoencoders.

Another type of ML is federated learning (also known as collaborative learning) that trains an algorithm across multiple decentralized devices holding local data, without exchanging the data. This approach stands in contrast to traditional centralized machine-learning techniques where all the local datasets are uploaded to one server, as well as to more classical decentralized approaches which often assume that local data samples are identically distributed. Federated learning enables multiple actors to build a common, robust machine learning model without sharing data, thus allowing to address critical issues such as data privacy, data security, data access rights and access to heterogeneous data.

In some examples, the AI model 8 can be trained continuously or periodically prior to performance of the inference operation by the processor 16. Then, during the inference operation, the patient specific input features, i.e. the 3D data D, provided to the AI model 8 may be propagated from an input layer, through one or more hidden layers, and ultimately to an output layer that corresponds to the system output, which is the 3D model M together with the classifier C.

For example, CT data of an area of inspection comprises 3D data of two inner organs of a patient. This 3D data D is propagated through the AI model 8 and the AI model 8 outputs to 3D models M of the two inner organs together with two classifiers C, a respective one for every model M. A respective one of the classifiers C is indicative of an anomaly of the inner organs, for example of nonconformity with standard anatomy, which is for example due to lesion. The classifier C can also indicate the degree of the anomaly.

During and/or subsequent to the inference operation, the 3D model M and the classifier C can be communicated to the user via the user interface, for example via the display 20, and/or automatically cause another surgical instruments to perform a desired action. For example, a surgical treatment can be suggested by the CDSS 2 based on the identified anomaly. The CDSS 2 can inform a clinician of the patent specific AI generated output. This can be a report of the suggested diagnosis and/or treatment option and corresponding AI generated confidence level.

The AI model 8 can be further trained to identify a plurality of anomalies in the 3D data D of the body part. It can be further trained to generate an anomaly list of identified anomalies, wherein the anomaly list is output via the user interface 10 at the display 20 together with the 3D model M and the classifier C. The anomalies can be prioritized on the anomaly list, for example depending on the severity of the anomaly. The AI model 8 can be further trained to perform an inference operation based on the 3D model M to generate the classifier C, in that it is additionally indicative of a relevance of the anomaly, based on which the prioritization on the anomaly list is performed, which can be the severity of the anomaly. The list can be displayed on a display 20 step by step with one anomaly displayed at a time. A region of the rendered 3D model M corresponding to the respective anomaly can be highlighted and/or zoomed-in. The user of the CDSS 2 can have the option to input additional information characterizing the respective anomaly. The data can be input via the user interface 18, which is for example a keyboard or mouse pointer, and can be stored for later use during, for example during preparation or execution of a surgical procedure.

According to another embodiment, the method of analyzing the 3D data D is applicable in the following scenario. The area of inspection, which is analyzed for example by CT or MRI, comprises a first area and a second area, which are not identical. The first and second area can be arranged not to overlap. This is however not always the case, even if the areas are not identical. For example, the first area can cover an inner organ, for example the liver. The second area can be the surrounding area, for example of the liver. There is an anatomical overlap and an overlap in image data. However, the areas are not identical. A first body part is located in the first area. A second body part is located in the second area. The 3D data D comprises information on the first and second body part. The first area and the first body part are labeled a subject and area of a surgical procedure. In other words, the first body part is for example the body part on which a surgical procedure shall be conducted. A first 3D model M is determined for the first body part and this 3D model M can be used to assist in the surgeon. In addition to this first model M, a second 3D model M is determined for the second body part. The second body part can for example be another inner organ of the patient, which is more distant or remote from the primary surgical field. According to further embodiments, a plurality of further body parts, which means a third, fourth and fifth body part can be in the 3D data D. Respective 3D models M are generated for these further body parts. The at least one second 3D model M of the second body part is analyzed by the AI model 8. Subsequently, the first 3D model M which serves as the basis for the surgical procedure and in addition to this, the at least one second 3D model M are output via the output interface 10 together with the classifier C for example at the display 20. If more than one second body part is inspected, more than one classifier C is output. The classifier C is indicative of at least one anomaly in the 3D data D of the at least one second body part. This information can assist the surgeon to potentially put focus on more remote organs or body parts that are in the primary surgical field.

The output can be a list. It can be output together with a prioritization information indicating a distance between the first body part (which is for example subject to the surgical treatment) and the at least one second body part (which is not the primary target of the surgical treatment). The prioritization information can indicate a distance between the first body part and an anomaly detected in the at least one second body part.

The database 12 can serve as computer-readable medium having instructions stored thereon causing a computer, for example the processor 16, which can be implemented in the CDSS 2 to execute the method of analyzing 3D data.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 2: clinical decision support system (CDSS)
- 4: input interface
- 6: 3D image-producing device
- 8: AI model
- 10: output interface
- 12: database
- 14: patient record
- 16: processing unit
- 18: user interface
- 20: display

- D: 3D data
- M: 3D model
- C: classifier

## Claims

1. A method of analyzing 3D data (D) acquired by a 3D image-producing procedure in an area of inspection comprising at least one body part of a patient's body, the method comprising the steps of:
- receiving the 3D data (D),
- determining a 3D model (M) of the body part from the 3D data (D) by inputting the 3D data (D) into an artificial intelligence model (8) to perform an inference operation based on the 3D data (D) to generate the 3D model (M) of the body part and a classifier (C), which is indicative of at least one anomaly in the 3D model (M) of the body part,
- outputting the 3D model (M) together with the classifier (C) at a user interface (10).

2. The method according to claim 1, wherein the artificial intelligence model (8) is a neural network, being trained on 3D training data, for which at least one of the following criteria applies:
a) the training data describes standard anatomy models of the at least one body part,
b) the training data describes standard anatomy models of the at least one body part having deviations from standard anatomy by less than a predetermined deviation threshold, wherein
a degree of anomaly indicated by the classifier (C) is a function of a deviation occurring when rendering the 3D model (M) of the body part against the training data.

3. The method according to claim 1 or 2, wherein the artificial intelligence model (8) is trained to perform the inference operation in that
a) the classifier (C) indicates top priority, if the 3D model (M) of the body part is not found in standard anatomy,
b) the classifier (C) indicates medium priority, if at least one anatomy deviation of the 3D model (M) from the standard anatomy is found, wherein this deviation is in a predetermined deviation interval,
c) the classifier (C) indicates low priority, if an anatomy deviation of the 3D model (M) from the standard anatomy is found, wherein this deviation is below a lower limit of the predetermined deviation interval.

4. The method according to any of the preceding claims, wherein the artificial intelligence model (8) further performs the inference operation based on the 3D data (D) to generate the classifier (C), in that it is additionally indicative of a type of the at least one anomaly in the 3D model (M) of the body part.

5. The method according to one of the preceding claims, wherein a plurality of anomalies are identified in the 3D model (M) of the body part, wherein the method further comprises the step of generating an anomaly list of identified anomalies, wherein the anomaly list is output via the user interface (10) together with the 3D model (M).

6. The method according to claim 5, wherein the anomalies are prioritized on the anomaly list, wherein in particular the artificial intelligence model (8) is further trained to perform an inference operation based on the 3D data (D) to generate the classifier (C), in that it is additionally indicative of a relevance of the anomaly, based on which the prioritization on the anomaly list is performed, and wherein further in particular the classifier (C) of every found anomaly comprises a relevance value.

7. The method according to claim 5 or 6, wherein the anomaly list is displayed step by step with one anomaly at a time, wherein a region of the 3D model (M) corresponding to the respective anomaly is highlighted and/or zoomed-in,
wherein in particular additional information characterizing the respective anomaly is added by user input.

8. The method according to any of the preceding claims, wherein the area of inspection comprises a first area and a second area, wherein the first and the second area are not identical, and a first body part is located in the first area and a second body part is located in the second area, and the 3D data (D) comprises information on the first and the second body part, wherein the first area and the first body part are labeled a subject and area of surgical procedure, and wherein a first 3D model (M) is determined for the first body part and a second 3D model (M) is determined for the second body part and at least the second 3D data of the second body part is input in the artificial intelligence model (8) to generate the second 3D model (M) of the second body part and the first and second 3D model (M) together with the classifier (C), which is indicative of at least one anomaly in the second 3D data (D) of the second body part are output via the user interface (10).

9. The method according to claim 8, further comprising the step of outputting a additional prioritization information together with the classifier (C), the prioritization information indicating a distance between the first body part and the second body part, in particular the prioritization information indicates a distance between the first body part and an anomaly detected in the second body part.

10. A computer-based clinical decision support system (2), comprising a processing unit (16), a user interface (18) and a display (20), configured to perform the method according to one of the preceding claims.

11. Computer program product comprising instructions which, when the program is executed by a computer (16), cause the computer (16) to perform the method according to one of claims 1 to 9.

12. Computer-readable medium (12) comprising instructions which, when executed by a computer (16), cause the computer (16) to perform the method according to one of the claims 1 to 9.
